# EUROPÄISCHE PATENTSCHRIFT

(11) **EP 2 648 807 B1**
(45) Veröffentlichungstag und Bekanntmachung des Hinweises auf die Patenterteilung: **24.01.2018**
(21) Anmeldenummer: 11796676.2
(22) Anmeldetag: 09.12.2011
(51) Int. Cl.: A61P 15/08, A61K 31/122, A61K 31/197, A61K 31/355, A61K 31/375, A61K 31/4188, A61K 31/4415, A61K 31/519, A61K 31/525, A61K 31/593, A61K 31/714, A61K 31/51, A23L 33/10, A23L 33/15, A23L 33/16, A61K 33/04, A61K 33/06, A61K 33/26, A61K 33/30, A61K 31/455, A61K 31/164

(54) **KOMBINATIONSPRÄPARAT ZUR VERBESSERUNG DER WEIBLICHEN FERTILITÄT**
COMBINATION PREPARATION FOR IMPROVING FEMALE FERTILITY
PRÉPARATION COMBINÉE DESTINÉE À AMÉLIORER LA FERTILITÉ CHEZ LA FEMME

(30) Priorität: 09.12.2010 AT 20372010; 13.09.2011 AT 13122011
(43) Veröffentlichungstag der Anmeldung: 16.10.2013
(73) Patentinhaber: Gonadosan GmbH, 9244 Niederuzwil (CH)
(72) Erfinder: WOGATZKY, Birgit, 88131 Lindau (DE); WOGATZKY, Johannes, 88131 Lindau (DE)
(74) Vertreter: Burger, Hannes
(86) Internationale Anmeldenummer: PCT/EP2011/072298
(87) Internationale Veröffentlichungsnummer: WO 2012/076680

(56) Entgegenhaltungen:
- EP-A1- 1 195 159
- WO-A1-2005/074950
- WO-A2-03/076600
- AU-A4- 2008 100 822
- DE-T2- 60 027 415
- M Bals-Pratsch: "Autoimmunthyreopathie und Kinderwunschbehandlung - Überlegungen zu einem empirischen Behandlungskonzept", Journal of Reproductive Medicine and Endocrinology - J. Reproduktionsmed. Endokrinol, 1 January 2005 (2005-01-01), pages 90-95, XP055249363, Retrieved from the Internet: URL:http://www.kup.at/kup/pdf/5208.pdf [retrieved on 2016-02-11]
- JOHANNES WOGATZKY: "Dietary Supplementation Improves Blastocyst Number and Ongoing Pregnancy Rate of IVF Patients with Hashimoto Thyroiditis", JOURNAL OF FOOD & NUTRITIONAL DISORDERS, vol. 02, no. 04, 1 January 2013 (2013-01-01), XP055249216, DOI: 10.4172/2324-9323.1000120

## Beschreibung

Die Erfindung betrifft ein Kombinationspräparat zur Verbesserung der Eizellqualität umfassend zumindest die Wirkstoffe C-Vitamine, E-Vitamine, B1-Vitamine, B2-Vitamine, B5-Vitamine, B6-Vitamine, B9-Vitamine, B12-Vitamine, D-Vitamine, zumindest eine Ubiquinon-10-Komponente und B7-Vitamine.

Immer mehr Paaren ist der Weg zum Wunschkind erschwert. In Industrieländern wie Deutschland und USA ist heute bereits eines von zehn Paaren ungewollt kinderlos und die Tendenz ist steigend. Eingeschränkte Fruchtbarkeit kann viele Ursachen haben. Das zunehmende Alter der Paare mit Kinderwunsch kann dabei ebenso eine Rolle spielen wie Umweltbelastung, verminderte Spermienqualität, beeinträchtigte Eizellen, ungesunde Lebensweise und anderes. Auch sogenannte "Lifestylefaktoren" stellen einen entscheidenden Faktor dar. Voraussetzung für die Fertilität ist bei Mann und Frau unter anderem eine ausreichende Versorgung des Körpers mit bestimmten Mikronährstoffen, Energiesubstraten und Vitaminen. Eine ausreichende Versorgung mit Vitaminen und gewissen Nährstoffen ist in unserer Zeit jedoch zunehmend schwierig. Bei erhöhten Belastungen im Alltag mit Stress, geringer Nahrungsmittelqualität und ungesunder Ernährung wird der menschliche Organismus oft unzureichend versorgt. Dadurch steht der Körper unter Dauerbelastung und das trifft besonders dort zu, wo die Zellen am empfindlichsten sind - im Bereich der Ei- und Samenzellen. Die männlichen Spermien werden träge, Eizellen "verpassen" ihren Eisprung und somit kann die Fruchtbarkeit unter Fehlernährung leiden. Es wurde bereits in mehreren Studien nachgewiesen, wie sehr die richtige Ernährung zur Erfüllung des Kinderwunsches beiträgt.

Während die Frau bereits mit den Eizellen geboren wird, werden die Spermien beim Mann ein Leben lang nachgebildet. Deshalb ist es für die Frau wichtig, ihren Körper auf eine Schwangerschaft vorzubereiten. Hierzu gibt es eine Vielzahl von Kombinationspräparten. In ihnen steckt eine Kombination von Mikronährstoffen, die für die gesunde Entwicklung der Eizellen notwendig sind.

So ist beispielsweise aus der US 2007/0104801 A1 ein Mittel zum Erhalt und zur Steigerung der weiblichen Fertilität bekannt, welches neben Glucosaminsulfat auch Selen, Folsäure, Vitamin B6, Vitamin B 12, Eisen, Zink, Magnesium, Grünen Tee, Mönchspfeffer und Hyaluronsäure enthält. Auch aus der US 6,569,857 B1 ist ein Nahrungsergänzungsmittel zur Steigerung der Konzeptionswahrscheinlichkeit und zur Unterstützung einer normalen Entwicklung eines Embryos bekannt, wobei neben den Vitaminen E, C, B1, B2, B3, B6, B9, B12 auch □-Carotin, Eisen, Kupfer, Zink, Calcium, Magnesium, Linolensäure und DHA enthalten sein können.

Die AU 2008/100822 A4 beschreibt in einem ersten Ausführungsbeispiel ein Nahrungsergänzungsmittel für Frauen zur Erleichterung der Konzeption umfassend Vitamine B1, B2, B3, B5, B6, C, D, E, B9, Eisen, Jod, Selen, Mangan, Magnesium Kupfer und Zink, essentielle Fettsäuren und Coenzym Q10. Gegebenenfalls kann Vitamin B 12 enthalten sein. Ferner wird ausgeführt, dass es aufgrund der hydrophilen und hydrophoben Eigenschaften der Wirkstoffe von Vorteil ist, nur ausgewählte Wirkstoffe in einer Formulierung zu kombinieren und mehrere Formulierungen zu verwenden, also z. B. eine Tablette und eine Kapsel. Es wird offenbart, welche Wirkstoffe in ein und derselben Formulierung kombiniert werden sollen. Hier wird insbesondere die Interaktion von Coenzym Q10 mit einigen Vitaminen und Mineralstoffen hervorgehoben, welche die Haltbarkeit von Coenzym Q10 beeinflussen und daher von Coenzym Q10 in der jeweils anderen Formulierung enthalten sein soll. Wird Biotin (Vitamin B7) verwendet, wird dieses bevorzugt in der ersten Formulierung, enthaltend Vitamine und Mineralstoffe, inkludiert.

Frauen mit spezifischen Erkrankungen, die einen Kinderwunsch tragen, weisen zudem besondere Bedürfnisse auf. So brauchen Frauen mit Autoimmunthyreoiditis eine speziell auf ihre Erkrankung zugeschnittene Behandlung. Bei der chronischen Autoimmunthyreoiditis handelt es sich um eine autoimmune Erkrankung der Schilddrüse. Dabei bildet das Immunsystem sogenannte Autoantikörper, welche Zellen der Schilddrüse angreifen und zerstören können. Es werden besonders häufig zwei klassische Formen beobachtet. Zum einen die sogenannte Hashimoto Thyreoiditis und zum anderen die Basedow Thyreoiditis. Die Unterscheidung dieser zwei Schilddrüsenerkrankungen kann dabei im Einzelfall sehr schwierig sein und es gibt sogar einige überlappende Formen.

Schätzungen zufolge hat jede sechste Frau eine Veranlagung für diese Autoimmunthyreoiditis und bei bis zu zehn Prozent von ihnen kommt es tatsächlich zur Bildung von Autoantikörpern. Die Ursachen und Auslöser einer Autoimmunthyreoiditis sind bis heute jedoch weitgehend unklar. Ein Zusammenhang mit genetischen Faktoren, Jodexzess, Veränderungen der Sexualhormone während der Schwangerschaft bzw. Menopause, Rauchen und Strahlen wird diskutiert.

M.Bals-Pratsch, et al. "Autoimmunthyreopathie und Kinderwunschbehandlung - Überlegungen zu einem empirischen Behandlungskonzept" J.Reproduktionsmed Endokrinol 2005; 2 (2): 90-5 beschreiben, dass die negativen Folgen der Antoimmunthyreopathie auf die reproduktive Funktion sehr effektiv behandelt werden, wenn die Schilddrüsenerkrankung als Autoimmunerkrankung betrachtet und parallel zur IVF-Behandlung neben einer Schilddrüsenhormongabe auch eine antiphlogistisch-immunsuppressive und antikoagulatorische Behandlung mit Prednisolon und Heparin begonnen wird.

Die Beschwerden sind vielfältig und variieren individuell. Für Frauen mit Kinderwunsch stehen jedoch Zyklusstörungen und ungewollte Kinderlosigkeit im Mittelpunkt. Auch das Risiko einer Fehlgeburt ist deutlich erhöht.

Vor und während einer Schwangerschaft sowohl gesunder Frauen als auch Frauen mit spezifischen Erkrankungen ist eine ausreichende und dem jeweiligen Stadium entsprechende Versorgung mit essentiellen Nährstoffen und Vitaminen für die Gesundheit des heranwachsenden Babys förderlich. Allerdings kann sich ein übermäßiger regelloser Vitaminkonsum in einigen Fällen sogar schädlich auf die Eizellqualität auswirken.

Aufgabe der vorliegenden Erfindung ist es daher ein Kombinationspräparat zur Verfügung zu stellen, welches die Eizellqualität verbessert, die Empfängnisbereitschaft erhöht und das Risiko einer Fehlgeburt reduziert.

Die Aufgabe der Erfindung wird jeweils eigenständig durch ein Kombinationspräparat zur Verwendung in der Behandlung weiblicher Infertilität bei Patientinnen mit Autoimmunthyreoiditis umfassend zumindest die Wirkstoffe C-Vitamin (Ascorbinsäure), E-Vitamine (Tocopherol), B1-Vitamin (Thiamin), B2-Vitamin (Riboflavin), B3-Vitamin, B5-Vitamine (Pantothensäure), B6-Vitamine (Pyridoxin), B7-Vitamin, B9-Vitamin (Folsäure), B12-Vitamine (Cobalamin), D-Vitamine (Calciferol, insbesondere Cholecalciferol) Zink, Magnesium, Eisen, Selen und/oder eine Selenverbindung und zumindest eine Ubiquinon-10-Komponente, wobei es jodfrei ist, gelöst. Von Vorteil dabei zeigt sich, dass durch die Kombination der oben genannten Wirkstoffe in einem Präparat deren synergistische Wirkung zur Geltung kommt. Durch die genannte Kombination der Wirkstoffe wird durch die gleichzeitige Freisetzung und somit Bioverfügbarkeit dem weiblichen Organismus ein Optimum an Wirkstoffen zur Verfügung gestellt, um die Eizellqualität zu verbessern, die Empfängnisbereitschaft zu erhöhen und das Risiko einer Fehlgeburt zu reduzieren. Die therapeutischen Wirkungen sind nachfolgend beschrieben: Vorteilhaft dabei erweist sich, dass durch die Supplementation einer Ubiquinon-10-Komponente die weiblichen Eierstöcke profitieren und somit sowohl die Eizell-lqualität als auch die Eizellreife verbessert werden. Die Ubiquinon-10-Komponente ist ein wichtiger Mikronährstoff für die Mitochondrien. Die weiblichen Keimzellen haben von allen Körperzellen mit am meisten Mitochondrien. Schädigungen der Mitochondrien führen einerseits zu einer geringeren Energiebereitstellung und andererseits zur Erhöhung von oxidativem Stress und somit zur Bildung von Sauerstoffradikalen in den Eizellen. Durch die zusätzliche Einnahme einer Ubiquinon-10-Komponente profitieren nachgewiesenermaßen die Eierstöcke. Sauerstoffradikale, die, wie bereits eingangs erwähnt, gebildet werden, greifen die Eizellmembran an. Ferner behindern sie die Mitochondrienfunktion und beeinflussen die Integrität der DNA und RNA sowie die Bildung und Funktion von zellulären Proteinen negativ. Viele Vitamine, insbesondere Vitamin C und E zeigen nachgewiesenermaßen hohes antioxidatives Potential. Oxidativer Stress verstärkt das autoimmune Geschehen, Antioxidantien hingegen wirken sich günstig auf den Krankheitsverlauf aus. Die Vitamine B6, B 12 und Folsäure des B-Komplexes wirken unter anderem regulierend auf den Homocystein-Stoffwechsel. Dies wirkt sich positiv auf die Gefäßfunktion aus und unterstützt somit eine ausreichende Durchblutung der Eierstöcke. Auch die Implantation des Embryos in der Frühschwangerschaft wird durch einen ausgeglichenen Homocystein-Stoffwechsel positiv beeinflusst. Insbesondere bei Patienten mit einer Folsäure-Stoffwechselstörung (MTHFR-Gen-Mutation) kann die Bildung von Mikrothromben bei der Implantation reduziert werden und somit auch die Frühabortrate durch die Vitamin B-Reihe reduziert werden. Ein Mangel an Vitamin B6 kann zu einer Erhöhung des Homocysteinspiegels führen. Dies kann zu Unfruchtbarkeit, erhöhter Abortneigung und Störung der Plazentafunktion beitragen. Folsäuremangel kann zu neurologischen Fehlbildungen (u.a. Spina bifida) und anderen Störungen beim Neugeborenen führen. Vitamine des D-Komplexes zeigen positive Wirkungen auf das Hormonsystem und auf den Phosphat-Haushalt und den Kalzium-Stoffwechsel, der in der Schwangerschaft (z.B. zum Aufbau des kindlichen Skelettsystems) eine wichtige Rolle spielt. Ferner ist das Vitamin-D-System wichtig für die Entwicklung und Funktion des Nerven- und Muskelsystems. B7-Vitamine spielen als prosthetische Gruppe von Enzymen im Stoffwechsel eine bedeutende Rolle, sind aber auch im Zellkern für die epigenetische Regulation der Genfunktion wichtig. Biotinmangel beeinträchtigt die Fruchtbarkeit und führt zu einer erhöhten Fehlbildungsrate. Patienten mit aktiver Autoimmunthyreoiditis weisen mit erhöhter Wahrscheinlichkeit einen Vitamin B 12 Mangel auf als gesunde Personen. Thiamin oder Vitamin B1 ist ein wasserlösliches Vitamin und ist insbesondere für die Funktion des Nervensystems unentbehrlich. Vitamin B2 oder Riboflavin dient als Vorstufe für wichtige Coenzyme. Dadurch nimmt es im Stoffwechsel eine zentrale Rolle ein. Vitamin B5 oder Pantothensäure besteht aus den beiden Bestandteilen Pantoinsäure und β-Alanin. Pantothensäure ist nötig für den Aufbau von Coenzym A, das eine wichtige Rolle im Stoffwechselgeschehen spielt. Es ist beteiligt am Auf- und Abbau von Kohlenhydraten, Fetten, Aminosäuren und an der Synthese von Cholesterin, das für die Bildung der Steroidhormone gebraucht wird. Beim Kombinationspräparat ist zudem von Vorteil, dass die spezielle Kombination der ausgewählten Wirkstoffe bessere Ergebnisse als im Vergleich zu den Einzelsubstanzen zeigt. Somit steht eine phasengerechte Versorgung der Frau mit Vitaminen, insbesondere vor, aber auch während der Schwangerschaft, zur Verfügung.

Das Kombinationspräparat wird vorzugsweise zur Behandlung weiblicher Infertilität, insbesondere für Frauen über 35 Jahre, verwendet, wodurch auch ein später Kinderwunsch unterstützt werden kann. Ubiquinon-10-Komponenten werden vom Körper unter anderem für die Energiegewinnung in den Mitochondrien benötigt. Insbesondere Frauen im fortgeschrittenen gebärfähigen Alter weisen vermehrte Schädigungen der Mitochondrien auf (Seifer et al, 2002), die einerseits zu einer herabgesetzten Energiebereitstellung und andererseits zur Erhöhung von oxidativem Stress in den Eizellen führen. Dies äußert sich in einer verringerten Eizellqualität und Beeinträchtig der frühen Embryonalentwicklung. In Studien konnte nachgewiesen werden, dass dies einer der Hauptgründe für den altersbedingten Rückgang der weiblichen Fertilität (Bentov et al, 2010) ist. Zur Förderung der mitochondrialen Funktion wird in dem Kombinationspräparat eine Ubiquinon Komponente eingesetzt. Ubiquinon-10-Komponenten sind sehr gute Antioxidantien, werden vom Körper jedoch auch für die Energiegewinnung in den Mitochondrien benötigt. Pignatti et al konnten 1980 zeigen, dass die Gewebekonzentration von Ubiquinon mit zunehmendem Alter abnimmt. Santos-Ocana bestätigten 2002 frühere Beobachtungen, die klar machen, dass Ubiquinon aber für die Energiebereitstellung in den Mitochondrien sehr wichtig ist. Die Ubiquinon-10-Komponente kann aus Ubiquinon-10 oder Ubichinol oder einer Mischung aus diesen Verbindungen bestehen, wobei Ubichinol rascher resorbiert werden kann und somit sofort verfügbar ist.

Vorteilhaft erweist sich die Ubiquinon-10-Komponente in Tagesdosen von 20 mg bis 100 mg, insbesondere 30 mg bis 50 mg, vorzugsweise 35 mg bis 40 mg, zu verabreichen, womit gewährleistet ist, dass ein angemessener Spiegel der Ubiquinon-10-Komponente im Organismus vorhanden ist und somit eine Unterstützung der Mitochondrienfunktion, insbesondere in den Eierstöcken, erfolgen kann. Dadurch wird eine gute Eizellqualität gefördert.

Die Ubiquinon-10-Komponente ist in Mengen von 2 bis 25 Gew.-%, insbesondere 5 bis 15 Gew.-%, vorzugsweise 8 bis 12 Gew.-%, bezogen auf das Gesamtgewicht der therapeutisch wirksamen Wirkstoffe des Kombinationspräparats enthalten, wodurch einerseits eine ausreichende Wirkung der Ubiquinon-10-Komponente gegeben ist, aber andererseits auch keine Gefahr zur Überdosierung besteht. Durch den gewählten Gewichtsanteil ist auch eine gute Verarbeitbarkeit in der Herstellung des Kombinationspräparats gegeben.

Durch die erfindungsgemäße Auswahl der Mengen der Wirkstoffe, wie C-Vitamine in Mengen von 4 bis 60 Gew.-%, insbesondere 10 bis 45 Gew.-%, vorzugsweise 19 bis 31 Gew.-%, E-Vitamine in Mengen von 1 bis 15 Gew.-%, insbesondere 2 bis 10 Gew.-%, vorzugsweise 3 bis 6 Gew.-%, B1-Vitamine in Mengen von 0,1 bis 3 Gew.-%, insbesondere 0,2 bis 2 Gew.-%, vorzugsweise 0,3 bis 0,9 Gew.-%, B2-Vitamine in Mengen von 0,1 bis 3 Gew.-%, insbesondere 0,2 bis 2 Gew.-%, vorzugsweise 0,3 bis 0,9 Gew.-%, B5-Vitamine in Mengen von 0,5 bis 10 Gew.-%, insbesondere 1 bis 5 Gew.-%, vorzugsweise 2 bis 3 Gew.-%, B6-Vitamine in Mengen von 0,1 bis 5 Gew.-%, insbesondere 0,5 bis 3 Gew.-%, vorzugsweise 0,8 bis 1,3 Gew.-%, B9-Vitamine in Mengen von 0,01 bis 1 Gew.-%, insbesondere 0,05 bis 0,5 Gew.-%, vorzugsweise 0,1 bis 0,3 Gew.-%, B 12-Vitamine in Mengen von 0,0003 bis 0,01 Gew.-%, insbesondere 0,0009 bis 0,005 Gew.-%, vorzugsweise 0,001 bis 0,008 Gew.-%, B7-Vitamine (Biotin) in Mengen von 0,001 bis 0,5 Gew.-%, insbesondere 0,007 bis 0,1 Gew.-%, vorzugsweise 0,01 bis 0,08, D-Vitamine in Mengen von 0,0002 bis 0,01 Gew.-%, insbesondere 0,0008 bis 0,008 Gew.-%, vorzugsweise 0,001 bis 0,005 Gew.-%, und eine Ubiquinon-10-Komponente in Mengen von 2 bis 25 Gew.-%, insbesondere 5 bis 15 Gew.-%, vorzugsweise 8 bis 12 Gew.-%, bezogen auf das Gesamtgewicht der therapeutisch wirksamen Wirkstoffe des Kombinationspräparats zeigen sich vorteilhafte Wirkungen bezüglich Eizellreife, Eizellqualität, Empfängnisbereitschaft, und Unterstützung der frühen Embryonalentwicklung. Die Wirksamkeit des Kombinationspräparats beruht nicht nur auf der antioxidativen Wirkung ausgewählter Einzelsubstanzen, sondern auch auf zahlreichen anderen Faktoren, wie z.B. der korrekten Zufuhr, insbesondere die zeitverzögerte Freisetzung von C-Vitaminen. Die Entwicklung und Reifung der Eizelle ist von der Anwesenheit einer gewissen Menge von Sauerstoffradikalen abhängig, weswegen eine vergleichsweise geringe Menge des antioxidativ wirksamen Vitamin C enthalten ist. Ein guter Schutz vor oxidativem Stress ist dennoch durch eine zeitverzögerte und nicht vollständig unmittelbar nach Einnahme des Kombinationspräparats einsetzende Freisetzung gegeben. Durch die Ausgewogenheit und die ausgewählte Zusammensetzung kann der gewünschte Effekt erzielt werden.

Vorteilhaft erweist sich auch die Zugabe von Niacin in Mengen von 1 bis 20 Gew.-%, insbesondere 3 bis 15 Gew.-%, vorzugsweise 5 bis 11 Gew.-%, Zink und/oder einer Zinkverbindung in Mengen von 0,2 bis 5 Gew.-%, insbesondere 1 bis 4,5 Gew.-%, vorzugsweise 1,5 bis 3,5 Gew.-%, Magnesium und/oder einer Magnesiumverbindung in Mengen von 10 bis 50 Gew.-%, insbesondere 15 bis 40 Gew.-%, vorzugsweise 25 bis 35 Gew.-%, Calcium und/oder eine Calciumverbindung, in Mengen von 5 bis 50 Gew.-%, insbesondere 15 bis 40 Gew.-%, vorzugsweise 20 bis 33 Gew.-%, und/oder Nikotinsäureamid in Mengen von 1 bis 15 Gew.-%, insbesondere 2 bis 10 Gew.-%, vorzugsweise 3 bis 6 Gew.-%, Eisen und/oder eine Eisenverbindung in Mengen von 0,5 bis 10 Gew.-%, insbesondere 1 bis 5 Gew.-%, vorzugsweise 1,5 bis 3 Gew.-%, bezogen auf das Gesamtgewicht der therapeutisch wirksamen Wirkstoffe des Kombinationspräparats, weil dadurch die vorab beschriebenen vorteilhaften Wirkungen sich kumulativ auf die Entwicklung der Eizelle äußern. Durch die beschriebene Zusammensetzung des Kombinationspräparats wird die Zusammenwirkung der Einzelsubstanzen untereinander optimiert und die Synergieeffekte zusätzlich verstärkt. Zudem kann eine ausgeglichene Wechselwirkung mit den anderen Substanzen erreicht werden. Durch Niacin (Vitamin B3) und Nikotinsäureamid wird beispielsweise die antioxidative Wirkung verstärkt und die Regeneration der DNA unterstützt. Für andere Substanzen, die während der Schwangerschaft vermehrt benötigt werden, wie beispielsweise Magnesium, wird bereits vor Eintritt der Schwangerschaft eine ausreichende Versorgung gegeben. In der Schwangerschaft kommt es oft zu einem Mangel an diesem Mineralstoff. Viele Schwangere nehmen die empfohlene Tagesdosis gar nicht zu sich. Hinzu kommen der erhöhte Magnesiumbedarf des Kindes und die erhöhte Magnesiumausscheidung (um bis zu 25%) der Mutter in der Schwangerschaft. Bei einer Unterversorgung mit Magnesium kann man häufig eine "muskuläre Übererregbarkeit" beobachten. Erste Anzeichen dafür sind beispielsweise Wadenkrämpfe. Diese muskulären Störungen können auch die Gebärmuttermuskulatur erfassen. Eine vorzeitige Wehentätigkeit und damit das Risiko einer Fehl- und Frühgeburt können die Folge sein. Eisen verbessert die Empfängnisbereitschaft der Frau. Der Bedarf an Eisen verdoppelt sich während einer Schwangerschaft. Eine nicht ausreichende Versorgung an diesem Mineralstoff führt zur sogenannten Schwangerschaftsanämie. Frauen mit

Eisenmangel weisen zudem eine verringerte Fertilität auf. Jod hat Einfluss auf den Hormonhaushalt.

Auch die Zugabe von weiteren Spurenelementen ausgewählt aus einer Gruppe umfassend Selen und/oder eine Selenverbindung in Mengen von 0,001 bis 0,05 Gew.-%, insbesondere 0,002 bis 0,01 Gew.-%, vorzugsweise 0,003 bis 0,005 Gew.-%, Kupfer und/oder eine Kupferverbindung in Mengen von 0,05 bis 3 Gew.-%, insbesondere 0,08 bis 1 Gew.-%, vorzugsweise 0,1 bis 0,5 Gew.-%, Molybdän und/oder eine Molybdänverbindung in Mengen von 0,0001 bis 0,5 Gew.-%, insbesondere 0,001 bis 0,1 Gew.-%, vorzugsweise 0,002 bis 0,05 Gew.-%, Chrom und/oder eine Chromverbindung in Mengen von 0,0001 bis 0,5 Gew.-%, insbesondere 0,001 bis 0,1 Gew.-%, vorzugsweise 0,002 bis 0,05 Gew.-%, Phosphor und/oder eine Phosphorverbindung in Mengen von 0,1 bis 30 Gew.-%, insbesondere 1 bis 20 Gew.-%, vorzugsweise 10 bis 15 Gew.-%, Mangan und/oder eine Manganverbindung in Mengen von 0,05 bis 3 Gew.-%, insbesondere 0,08 bis 1 Gew.-%, vorzugsweise 0,1 bis 0,5 Gew.-%, bezogen auf das Gesamtgewicht der therapeutisch wirksamen Wirkstoffe des Kombinationspräparats kann die vorteilhaften Wirkungen auf die Eizelle, insbesondere die Eizellqualität weiter verbessern. Spurenelemente sind für den Menschen essentielle Nährstoffe, deren Fehlen schwere Mangelerscheinungen hervorrufen kann. Eine tägliche Seleneinnahme in hoher Dosierung kann eine Verbesserung der Beschwerden bei Hashimoto-Thyreoiditis bewirken (Gärtner et al., 2002, Duntas et al., 2003). Für diesen positiven Effekt scheinen zwei Eigenschaften von Selen verantwortlich zu sein. Erstens wird es von dem selenabhängigen Enzym Jodthyronin-Dejodase für die Umwandlung des Schilddrüsenhormons T4 (Speicherform) in die stoffwechselaktive Form T3 benötigt. Bei einem zu niedrigen Selenlevel kann keine ausreichende Menge T3 aus dem vorhandenen T4 hergestellt werden. Außerdem kann Selen als Bestandteil des Enzyms Glutathionperoxidase die spezifischen Antikörper verantwortlich für Autoimmunthyreoiditis senken. Es wirkt hier als Antioxidans und fängt schädliche Radikale ab, die in der Schilddrüse als Nebenprodukt bei der Bildung der Schilddrüsenhormone entstehen. Dadurch verbessert es die Funktionsfähigkeit des Immunsystems (Gärtner et al., 2002, Turker et al., 2006). Die Böden in Europa sind sehr selenarm, sodass in Pflanzen, die auf diesen Böden wachsen, und damit in der Nahrung, nur wenig Selen vorhanden ist. Bei entzündlichen Erkrankungen wie der Hashimoto-Thyreoiditis besteht außerdem ein erhöhter Bedarf, der über die normale Ernährung erst recht nicht mehr gedeckt werden kann.

Vorteilhaft erweist sich auch die Beimengung von Vorstufen der A-Vitamine, insbesondere α- und β-Carotin, α- und β-Cryptoxanthin und Lycopin, in Mengen von 0,0001 bis 0,05 Gew.-%, insbesondere 0,0002 bis 0,001 Gew.-%, vorzugsweise 0,0003 bis 0,0006 Gew.-%, und/oder K-Vitaminen, insbesondere K1, K2, K3 in Mengen von 0,05 bis 3 Gew.-%, insbesondere 0,08 bis 1 Gew.-%, vorzugsweise 0,1 bis 0,5 Gew.-%, bezogen auf das Gesamtgewicht der therapeutisch wirksamen Wirkstoffe des Kombinationspräparats, weil durch Östrogeneinnahme die Verwertung von A-Vitaminen gestört sein kann und durch Beimengung von A-Vitaminen zum Kombinationspräparat dennoch für eine ausreichende Versorgung von A-Vitaminen gesorgt ist und damit ihre positiven Wirkungen auf das Wachstum ausüben kann.

Sind Omega-3-Fettsäuren, insbesondere DHA und/oder EPA, in Mengen von 2 bis 70 Gew.-%, insbesondere 10 bis 50 Gew.-%, vorzugsweise 20 bis 40 Gew.-%, bezogen auf das Gesamtgewicht der therapeutisch wirksamen Wirkstoffe des Kombinationspräparats enthalten, wird bereits vor Eintritt der Schwangerschaft gewährleistet, dass eine ausreichende Versorgung gegeben ist und somit der erhöhte Bedarf während der Schwangerschaft bereits zu Beginn abgedeckt ist. Eine Gabe von Omega -3-Fettsäuren wird jedoch erst ab dem 3. Schwangerschaftsmonat angeraten und kann dann auch die neurologische Entwicklung des Feten fördern.

Durch die Zugabe von Probiotika in Mengen von 0,1 bis 10 Gew.-%, insbesondere 0,5 bis 5 Gew.-%, vorzugsweise 1 bis 3 Gew.-%, bezogen auf das Gesamtgewicht der therapeutisch wirksamen Wirkstoffe des Kombinationspräparats wird erreicht, dass das Mukosaimmunsystem stimuliert bzw. moduliert wird. Auch vaginale Infekte können durch Probiotika positiv beeinflusst werden und somit ein günstigeres Milieu für eine Schwangerschaft schaffen.

Im Sinne der Erfindung werden unter pre-A-, B-, C-, D- und E-Vitaminen alle zu diesen Vitaminen bekannten Vertreter verstanden. So sollen beispielsweise durch E-Vitamine Tocopherole, Tocotrienole und Tocomonoenole umfasst werden. Durch die Bezeichnung D-Vitamine sind insbesondere Vitamin D3, 25(OH) Vitamin D3 und Calcitriol umfasst. Dies sei nur beispielhaft erwähnt und gilt auch für die anderen in der Erfindung genannten Vitamine. Selbiges wie vorab beschrieben gilt auch für die anderen in der Erfindung genannten Wirkstoffe, wo auch deren Derivate bzw. Salze mitumfasst sein sollen, die dem Körper bioverfügbar zur Disposition stehen.

Die vorliegende Erfindung beschreibt unter anderem die positive Wirkung von Ubiquinon-10-Komponenten auf die Eizelle, insbesondere die Reifung der Eizelle und die frühe Embryonalentwicklung und Implantation. Eine Ubiquinon-10-Komponente wird vom Körper unter anderem für die Energiegewinnung in den Mitochondrien benötigt. Sie ist ein wichtiger Mikronährstoff für die Unterstützung der mitochondrialen Gesundheit. Von allen Körperzellen haben die weiblichen Eizellen die meisten Mitochondrien. Insbesondere Frauen im fortgeschrittenen gebärfähigen Alter weisen jedoch vermehrte Schädigungen der Mitochondrien auf, die einerseits zu einer herabgesetzten Energiebereitstellung und andererseits zur Erhöhung von oxidativem Stress in den Eizellen führen. Dies äußert sich in einer verringerten Eizellqualität. Dies ist ein Hauptgrund für den altersbedingten Rückgang der weiblichen Fruchtbarkeit. Die Ubiquinon-10-Komponenten sinken mit zunehmendem Alter ab und dies erklärt den förderlichen Einfluss einer Ubiquinon Supplementierung bei Frauen über 35 mit Kinderwunsch. Ubichinon-10-Komponenten sind zudem ein wichtiges Antioxidans.

Verschiedene Prozesse wie Umweltverschmutzung, Fehlernährung, Strahlung und andere Belastungen führen im Körper zu oxidativem Stress. Darunter versteht man die Schädigung der Zellen durch Sauerstoffradikale und andere reaktive Sauerstoffspezies. Insbesondere die Keimzellen, Spermien und Eizellen, sind hier besonders anfällig. Ebenso wie die männliche Fruchtbarkeit wird auch die weibliche Fertilität durch oxidativen Stress negativ beeinflusst. Der normale altersabhängige Rückgang der Fertilität der Frau steht unter anderem in Zusammenhang mit oxidativen Schäden. Dies konnte durch wissenschaftliche Studien zweifelsfrei nachgewiesen werden. Normalerweise hat der Körper eine Reihe von eigenen Abwehrmechanismen, unter anderem körpereigene Antioxidantien - doch diese sind, insbesondere im fortgeschrittenen Lebensalter, nicht immer ausreichend vorhanden.

Das erfindungsgemäße Kombinationspräparat ist speziell für die Bedürfnisse des weiblichen Körpers bei Kinderwunsch und Schwangerschaft entwickelt. Es kann sowohl ein Nahrungsergänzungsmittel, ein diätetisches Lebensmittel für besondere medizinische Zwecke (ergänzende bilanzierte Diät), als auch ein Arzneimittel sein. Dadurch ist für den Anwender gewährleistet, dass bei der Zulassung als Arzneimittel bzw. bilanzierte Diät das Präparat zumindest eine klinische Studie durchlaufen hat und bei der Zulassung als Nahrungsergänzungsmittel ein einfacher und gegebenenfalls diskreter Zugang zum Präparat gegeben ist.

Durch das erfindungsgemäße Kombinationspräparat werden Eizellen vor schädlichen Einflüssen durch oxidativen Stress geschützt und zugleich mit den für die Entwicklung notwendigen Nähr- und Mineralstoffen versorgt, um ihnen eine bestmögliche Entwicklung zu ermöglichen.

Ferner zeigt das erfindungsgemäße Kombinationspräparat eine speziell abgestimmte Mischung von Antioxidantien, teilweise in Depotform, und anderer für die Fortpflanzungszellen wichtiger Nähr- und Mineralstoffe. So wird beispielsweise Vitamin C als Retardform beigemengt, wodurch dem Körper zeitversetzt über den ganzen Tag verteilt ausreichend Vitamin C zur Verfügung steht und nicht sofort nach Aufnahme des Kombinationspräparats die gesamte Menge im Blutkreislauf zirkuliert. Die retardierte Freisetzung von Vitamin C ist besonders von Vorteil, weil ein Überschuss von Antioxidantien auch schädlich wirken kann. Aus dem Stand der Technik sind Zubereitungen für eine retardierte Freisetzung von Wirkstoffen beschrieben. Beispielsweise kann Vitamin C mit Hydroxypropylmethylcellulose beschichtet sein. Selbstverständlich können auch andere aus dem Stand der Technik bekannte Methoden für die zeitversetzte Freisetzung von Vitamin C angewendet werden. Für fertilitätssteigernde Produkte ist die Verwendung der Retardform von Vitamin C aus dem Stand der Technik allerdings bisher nicht bekannt.

Die Wirkmechanismen der einzelnen Substanzen im erfindungsgemäßen Kombinationspräparat sind komplex. Neben Nährstoffgabe und Enzyminduktion ist ein wesentlicher Wirkmechanismus die gezielte Reduktion der Sauerstoffradikale.

Alle Einzelstoffe in dem erfindungsgemäßen Kombinationspräparat haben in Untersuchungen gezeigt, dass eine zusätzliche Einnahme von den im erfindungsgemäßen Kombinationspräparat abgestimmten Antioxidantien bei Frauen zu einer deutlichen Verbesserung ihrer Fruchtbarkeit führt.

Die vorab beschriebenen Mechanismen funktionieren vorzugsweise in Kombination der Wirkstoffe und durch Unterstützung mehrerer Antioxidativa. Weitere Substanzen wie Zink, das an fast allen biochemischen Prozessen im Körper beteiligt ist und Bestandteil antioxidativ wirksamer Enzyme ist, unterstützen die Wirkung des erfindungsgemäßen Kombinationspräparats. Zink kann im Körper nicht gespeichert werden, es muss regelmäßig von außen zugeführt werden. Aufgrund von falschen Ernährungsgewohnheiten ist Zinkmangel auch in westlichen Ländern nicht selten. Bei der Hashimoto-Thyreoiditis ist die zeitlich begrenzte Zufuhr von Zink sinnvoll, da diesem Spurenelement eine entscheidende Rolle im Immunsystem zukommt und Zink zudem eine zentrale Bedeutung für den Stoffwechsel der Schilddrüsenhormone hat. Verschiedene Tierversuche an Ratten zeigten, dass eine Hypothyreose im Vergleich zu einer ausgeglichenen Schilddrüsenstoffwechsellage mit einem schlechteren Zinkstatus einhergeht.

Durch den synergistischen Effekt der einzelnen Wirkstoffe in den ausgewählten Mengenbereichen kann die Wirksamkeit bezüglich Eizellqualität und Eizellreife des erfindungsgemäßen Kombinationspräparats erzielt werden.

Das Kombinationspräparat wird vorzugsweise oral eingenommen. Das Präparat kann sowohl als Human- als auch Veterinärprodukt verwendet werden.

In der nachfolgenden Tabelle sind die Mengenbereiche der empfohlenen Tagesdosis der einzelnen für die Erfindung relevanten Wirkstoffe angegeben. Jedenfalls enthält das Kombinationspräparat die Wirkstoffe C-Vitamine, E-Vitamine, B1-Vitamine, B2-Vitamine, B5-Vitamine, B6-Vitamine, B9-Vitamine, B 12-Vitamine, D-Vitamine, zumindest eine Ubiquinon-10-Komponente und B7-Vitamine. Bei den weiteren angeführten Wirkstoffen, die optional enthalten sein können, sind jeweils Unter- und Obergrenzen angeführt. Vorzugsweise wird das erfindungsgemäße Kombinationspräparat als eine Kapsel pro Tag eingenommen. Es können allerdings die Wirkstoffmengen so gewählt werden, dass zwei Kapseln pro Tag erforderlich sind, um die empfohlene Dosierung zu erreichen.

| **Wirkstoff** | **empfohlener Mengenbereich in mg pro Tag** |
|---|---|
| Vitamin C | 80-120 |
| Vitamin E | 10-30 |
| Vitamin B1 | 1 - 3 |
| Vitamin B2 | 1,5 - 3 |
| Vitamin B5 | 6 - 18 |
| Vitamin B6 | 3 - 5 |
| Vitamin B 12 | 0,005 - 0,008 |
| Vitamin B9 | 0,6 - 0,8 |
| Vitamin D | 0,003 - 0,006 |
| Vitamin B7 | 0,1 - 0,2 |
| Ubiquinon-10 Komponente | 30 - 50 |
| Niacin | 15 - 40 |
| Zink | 5 - 20 |
| Magnesium | 100 - 300 |
| Jod | 0- 0,15 |
| Eisen | 5 - 15 |
| Selen | 0,01 - 0,2 |
| Calcium | 100 - 400 |
| Omega-3-Fettsäuren | 200 - 500 |
| Vitamin A | 0,01 - 0,5 |
| Nicotinsäure-amid | 15 - 36 |
| Vitamin K | 0,01 - 0,1 |
| Kupfer | 0,5 - 3 |
| Molybdän | 0,01 - 0,1 |
| Chrom | 0,01 - 0,1 |
| □ -Carotin | 1 - 5 |
| Probiotika | 0,1 - 10 |
| Phosphor | 50 - 150 |
| Mangan | 0,5 - 3 |

Im nachfolgenden sind Ausführungsbeispiele zur Zusammensetzung der therapeutisch wirksamen Wirkstoffe des erfindungsgemäßen Kombinationspräparats angeführt, das die vorteilhaften Wirkungen erzielt.

Es sind auch Exzipienten, wie Hilfsstoffe, Füllstoffe, Trägerstoffe, Bindemittel, etc., welche zur technischen Verarbeitbarkeit des erfindungsgemäßen Kombinationspräparats erforderlich sind, enthalten. Diese Exzipienten haben jedoch keine therapeutische Wirksamkeit und keine positiven Effekte auf die Eizellqualität und sind dementsprechend auch keine therapeutisch wirksamen Wirkstoffe im Sinne der Erfindung.
Die nachfolgenden Tabellen zeigen 20 Ausführungsbeispiele unter Angabe der absoluten Zahlen der therapeutisch wirksamen Wirkstoffe, wobei alle bis auf Beispiel 13 Referenzbeispiele sind.

| **Wirkstoff** | **1** | **2** | **3** | **4** | **5** | **6** | **7** | **8** | **9** | **10** |
|---|---|---|---|---|---|---|---|---|---|---|
| Vitamin C | - | 80 | 100 | 120 | 95 | 80 | 100 | 120 | 80 | 100 |
| Vitamin E | - | 10 | 20 | 30 | 25 | 10 | 20 | 30 | 10 | 20 |
| Vitamin B1 | - | 1 | 3 | 3 | 2,5 | 1 | 3 | 3 | 1 | 3 |
| Vitamin B2 | - | 1,5 | 3 | 3 | 2 | 1,5 | 3 | 3 | 1,5 | 3 |
| Vitamin B5 | - | 6 | 12 | 18 | 15 | 6 | 12 | 18 | 6 | 12 |
| Vitamin B6 | - | 3 | 4 | 5 | 3 | 3 | 4 | 5 | 3 | 4 |
| Vitamin B12 | - | 0,005 | 0,007 | 0,008 | 0,006 | 0,005 | 0,007 | 0,008 | 0,005 | 0,007 |
| Vitamin B9 | - | 0,6 | 0,8 | 0,8 | 0,7 | 0,6 | 0,8 | 0,8 | 0,6 | 0,8 |
| Vitamin D | - | 0,003 | 0,005 | 0,006 | 0,004 | 0,003 | 0,005 | 0,006 | 0,003 | 0,005 |
| Niacin | - | - | - | - | - | 15 | 35 | 40 | 15 | 35 |
| Vitamin B7 | - | - | - | - | - | 0,1 | 0,2 | 0,2 | 0,1 | 0,2 |
| Zink | - | - | - | - | 14 | 5 | 5 | 20 | 5 | 5 |
| Magnesium | - | - | - | - | - | 100 | 100 | 300 | 100 | 100 |
| Jod | - | - | - | - | 0,05 | 0 | 0,15 | 0,15 | 0 | 0,15 |
| Eisen | - | - | - | - | - | 5 | 7,5 | 15 | 5 | 7,5 |
| Ubiquinon-10 Komponente | 50 | 30 | 35 | 50 | 40 | 30 | 35 | 50 | 30 | 35 |
| Selen | - | - | - | - | - | - | - | - | - | - |
| Calcium | - | - | - | - | 250 | | - | - | 100 | 125 |
| Omega-3-Fettsäuren | - | - | - | - | - | - | - | - | - | - |
| Vitamin A | - | - | - | - | - | - | - | - | - | - |
| Nicotinsäure- amid | - | - | - | - | - | - | - | - | 10 | 20 |
| Vitamin K | - | - | - | - | - | - | - | - | - | - |
| Kupfer | - | - | - | - | - | - | - | - | - | - |
| Molybdän | - | - | - | - | 0,4 | - | - | - | - | - |
| Chrom | - | - | - | - | - | - | - | - | - | - |
| □-Carotin | - | - | - | - | - | - | - | - | - | - |
| Probiotika | - | - | - | - | 4 | - | - | - | - | - |
| Phosphor | - | - | - | - | - | - | - | - | - | - |
| Mangan | - | - | - | - | - | - | - | - | - | - |
| Füll- und Trägerstoffe | ✔ | ✔ | ✔ | ✔ | ✔ | ✔ | ✔ | ✔ | ✔ | ✔ |

| **Wirkstoff** | **11** | **12** | **13** | **14** | **15** | **16** | **17** | **18** | **19** | **20** |
|---|---|---|---|---|---|---|---|---|---|---|
| Vitamin C | 120 | 96 | 80 | 100 | 120 | 85 | 105 | 100 | 115 | 95 |
| Vitamin E | 30 | 23 | 10 | 20 | 30 | 11 | 30 | 20 | 21 | 15 |
| Vitamin B1 | 3 | 2 | 1 | 3 | 3 | 1 | 3 | 2 | 2 | 1,5 |
| Vitamin B2 | 3 | 2 | 1,5 | 3 | 3 | 1,8 | 2,9 | 1,7 | 2,4 | 2,9 |
| Vitamin B5 | 18 | 10 | 6 | 12 | 18 | 15 | 10 | 6 | 9 | 18 |
| Vitamin B6 | 5 | 5 | 3 | 4 | 5 | 5 | 3,5 | 3 | 4,4 | 4 |
| Vitamin B12 | 0,008 | 0,008 | 0,005 | 0,007 | 0,008 | 0,007 | 0,008 | 0,006 | 0,005 | 0,005 |
| Vitamin B9 | 0,8 | 0,6 | 0,6 | 0,8 | 0,8 | 0,7 | 0,8 | 0,7 | 0,6 | 0,6 |
| Vitamin D | 0,006 | 0,004 | 0,003 | 0,005 | 0,006 | 0,005 | 0,003 | 0,006 | 0,004 | 0,003 |
| Niacin | 40 | - | 15 | 35 | 40 | 22 | - | 38 | 34 | 17 |
| Vitamin B7 | 0,2 | - | 0,1 | 0,2 | 0,2 | 0,15 | 0,11 | 0,12 | - | - |
| Zink | 20 | - | 5 | 5 | 20 | - | - | - | - | - |
| Magnesium | 300 | - | 100 | 100 | 300 | 100 | 120 | 150 | 230 | - |
| Jod | 0,15 | - | 0 | 0,15 | 0,15 | - | - | - | - | - |
| Eisen | 15 | - | 5 | 7,5 | 15 | - | - | - | - | - |
| Ubiquinon-10 Komponente | 50 | 42 | 30 | 35 | 50 | 50 | 40 | 32 | 46 | - |
| Selen | - | - | 0,01 | 0,03 | 0,1 | - | 0,08 | - | - | - |
| Calcium | 500 | 120 | 100 | 120 | 500 | - | - | - | - | - |
| Omega-3-Fettsäuren | - | - | 20 | 400 | 1000 | - | - | - | 300 | - |
| Vitamin A | - | 0,001 | 0,001 | 0,001 | 0,01 | - | - | - | - | - |
| Nicotinsäureamid | 50 | 30 | 10 | 20 | 50 | - | - | - | - | - |
| Vitamin K | - | - | 0,5 | 1 | 10 | - | 4 | - | - | - |
| Kupfer | - | - | 0,5 | 1 | 10 | - | - | 0,5 | - | - |
| Molybdän | - | - | 0,01 | 0,08 | 0,5 | - | - | - | 0,1 | - |
| Chrom | - | 0,1 | 0,01 | 0,06 | 0,5 | 0,09 | - | - | - | - |
| □-Carotin | - | - | 0,5 | 2 | 10 | - | - | 5 | - | - |
| Probiotika | - | - | 0 | 1 | 5 | - | - | - | - | - |
| Phosphor | - | - | 1 | 125 | 200 | 12 | - | - | - | - |
| Mangan | - | - | 0,1 | 1 | 10 | - | - | 0,9 | - | - |
| Füll- und Trägerstoffe | ✔ | ✔ | ✔ | ✔ | ✔ | ✔ | ✔ | ✔ | ✔ | ✔ |

In Versuchen konnte nachgewiesen werden, dass die Ausführungsbeispiele 7 und 10 besonders gute Wirksamkeit zeigen. So konnte beispielsweise mit der Zusammensetzung gemäß Ausführungsbeispiel 7 im Vergleich zu einem reinen Folsäurepräparat bzw. einem einfachen Multivitaminpräparat eine um 10 % höhere Anzahl an positiven Schwangerschaftstests nach reproduktionsmedizinischer Behandlung erreicht werden. Dieses Ergebnis bestätigt sich bei Wiederholung sowohl mit Frauen unter 35 Jahren als auch Frauen über 35 Jahren. Bei Frauen über 35 Jahren kann auch sowohl die Anzahl der gewonnenen Eizellen nach einem Stimulationszyklus als auch die Anzahl für Metaphase II Eizellen sowie die Anzahl erfolgreich befruchteter Eizellen um annähernd 10 % erhöht werden. Auch mit einer Zusammensetzung gemäß Ausführungsbeispiel 10 kann nachgewiesen werden, dass es durch eine Förderung der Eizellreifung zu einer um ca. 10 % gesteigerten Eizellmenge nach Stimulation im Rahmen einer reproduktionsmedizinischen Behandlung und zu einer um ca. 10 % erhöhten Befruchtungsrate kommt. Generell wurde nachgewiesen, dass mit dem Kombinationspräparat die Eizellqualität bei über 90 % der Frauen, insbesondere von über 35- jährigen signifikant verbessert wird.

In der unten stehenden Tabelle ist beispielhaft die Zusammensetzung der Wirkstoffe in absoluten und relativen Zahlen eines Referenzbeispiels eines Kombinationspräparats (Ausführungsbeispiel 10 und 7) beschrieben.

| **Wirkstoff** | **Menge in mg** | **Gew.-%** | **Menge in mg** | **Gew.-%** |
|---|---|---|---|---|
| Vitamin C | 100 | 21,24666958 | 100 | 30,70668362 |
| Vitamin E | 20 | 4,249333917 | 20 | 6,141336723 |
| Vitamin B1 (Thiamin) | 3 | 0,637400088 | 3 | 0,921200509 |
| Vitamin B2 (Riboflavin) | 3 | 0,637400088 | 3 | 0,921200509 |
| Pantothensäure B5 | 12 | 2,54960035 | 12 | 3,684802034 |
| Vitamin B6 (Pyridoxin) | 4 | 0,849866783 | 4 | 1,228267345 |
| Vitamin B12 (Cobalamin) | 0,007 | 0,001487267 | 0,007 | 0,002149468 |
| Folsäure | 0,8 | 0,169973357 | 0,8 | 0,245653469 |
| Vitamin D | 0,005 | 0,001062333 | 0,005 | 0,001535334 |
| Niacin (Nicotinsäure) | 35 | 7,436334355 | 35 | 10,74733927 |
| Biotin | 0,2 | 0,042493339 | 0,2 | 0,061413367 |
| Zink | 5 | 1,062333479 | 5 | 1,535334181 |
| Magnesium | 100 | 21,24666958 | 100 | 30,70668362 |
| Jod | 0,15 | 0,031870004 | 0,15 | 0,046060025 |
| Eisen | 7,5 | 1,593500219 | 7,5 | 2,303001271 |
| Ubichinon-10-Komponente (Coenzym Q10) | 35 | 7,436334355 | 35 | 10,74733927 |
| Calcium | 125 | 26,55833698 | - | - |
| Nicotinsäureamid | 20 | 4,249333917 | - | - |

In einer Alternative besteht das Kombinationspräparat aus Magnesiumoxid, L-Ascorbinsäure, Coenzym Q10 (7,5%), Kieselsäure-Gel, Eisencitrat (5,2 %), D-alpha-Tocopherylacetat, Nicotinamid, Calcium-D-pantothenat, Nicotinsäure, Zinkoxid, Pyridoxin-HCl, Thiamin-HCl, Maltodextrin, Riboflavin, Verdickungsmittel Gummi Arabicum, Natriumcitrat, Saccharose, Maisstärke, Säuerungsmittel Citronensäure, Pteroylmonoglutaminsäure (0,17 %), Biotin, Antioxidationsmittel alpha-Tocopherol, Stabilisator Tricalciumphosphat, Farbstoff Titandioxid, Farbstoff Gelborange S, Cholecalciferol, Cyanocobalamin. Die Kapselhülle ist vorzugsweise aus Cellulose gebildet bzw. überzogen.
Die positive Wirkung des erfindungsgemäßen Kombinationspräparats kann sowohl in Vorbereitung für eine natürliche Schwangerschaft als auch in Vorbereitung für eine Insemination, In vitro Fertilisierung als auch Intracytoplasmatische Spermieninjektion genutzt werden.
In einer Alternative kann auch ein Kombinationspräparat hergestellt werden, welches keine Ubichinon-10-Komponente enthält. Dieses Kombinationspräparat umfasst jedenfalls C-Vitamine, E- Vitamine, B1-Vitamine, B2-Vitamine, B5-Vitamine, B6-Vitamine, B12-Vitamine, B9-Vitamine, D-Vitamine und Niacin und gegebenenfalls B7-Vitamine, Zink, Magnesium, Jod, Eisen und/oder Calcium. Die Mengenbereiche der Wirkstoffe sind gleich wie vorab beschrieben.
Nachfolgend finden sich Ausführungsbeispiele als Referenzbeispiele zu dieser Ubiquinon-10-Komponentefreien Alternative, wobei die Mengenangaben in mg/Kapsel erfolgen.

| **Wirkstoff** | **21** | **22** | **23** |
|---|---|---|---|
| Vitamin C | 100 | 100 | 100 |
| Vitamin E | 20 | 20 | 20 |
| Vitamin B1 | 3 | 3 | 3 |
| Vitamin B2 | 3 | 3 | 3 |
| Vitamin B5 | 12 | 12 | 12 |
| Vitamin B6 | 4 | 4 | 4 |
| Vitamin B12 | 0,007 | 0,007 | 0,007 |
| Vitamin B9 | 0,8 | 0,8 | 0,8 |
| Vitamin D3 | 0,005 | 0,005 | 0,005 |
| Niacin | 15 | - | 15 |
| Vitamin B7 | 0,2 | - | 0,2 |
| Zink | 5 | - | 5 |
| Magnesium | 100 | - | 100 |
| Jod | 0,15 | - | 0,15 |
| Eisen | 7,5 | - | 15 |
| Nicotinamid | 20 | - | 20 |
| Calcium | 125 | - | - |

In einer Weiterbildung dieser Alternative können auch noch die Wirkstoffe wie Selen, Omega-3-Fettsäuren, A-Vitamine, K-Vitamine, Kupfer, Molybdän, Chrom, b-Carotin, Phosphor, Mangan und/oder Probiotika in den vorab beschriebenen Mengenbereichen enthalten sein.

Auch diese Alternative zeigt eine gute Wirkung auf die Eizellqualität und die Eizellreife, wenngleich der Erfolg unter Verwendung einer Ubiquinon-Q10-Komponente insbesondere bei Frauen über 35 größer ist.

In einer weiteren Alternative enthält das Kombinationspräparat Wirkstoffe, die auf die speziellen Bedürfnisse von Frauen mit Kinderwunsch und aktiver Autoimmunthyreoiditis angepasst sind. Diese Ausführungsvariante des Kombinationspräparats bereitet den Körper durch seine besondere Zusammensetzung auf eine bevorstehende Schwangerschaft vor und fördert die Fruchtbarkeit. Eine Behandlung mit Schilddrüsenhormonen kann in vielen Fällen eine Schwangerschaft ermöglichen. Ergänzend hierzu können bestimmte Vitamine und Vitalstoffe den Krankheitsverlauf positiv beeinflussen. Wichtig ist in diesem Zusammenhang jedoch, dass die Zufuhr von Iod im Falle einer aktiven Entzündung in jedem Fall vermieden werden sollte, weil es zu einer deutlichen Verschlechterung der Situation führen kann. Daher benötigen Frauen mit Autoimmunthyreoiditis besondere, jodfreie Präparate. Zudem verbessert eine tägliche vermehrte Seleneinnahme die Beschwerden bei Hashimoto-Thyreoiditis. Patienten mit Autoimmunthyreoiditis zeigen erhöhte Werte für oxidativen Stress sowie verringerte Antioxidantienwerte. Eine zusätzliche Versorgung des Körpers mit Antioxidantien ist daher notwendig und beeinflusst die Erkrankung in ihrem Verlauf positiv. Gleichzeitig werden auch die Eizellen vor den schädlichen Auswirkungen von oxidativem Stress geschützt. Patienten mit Autoimmunthyreoiditis leiden vermehrt an Vitamin B12-Mangel. Dies ist insbesondere im Falle eines Kinderwunsches problematisch, da Vitamin B 12 neben seiner Bedeutung für die Zellteilung zusammen mit anderen Vitaminen der B-Gruppe, insbesondere Vitamin B6 und Folsäure, unentbehrlich für die Regulation des Homocysteinstoffwechsels ist.

Nachfolgend finden sich Ausführungsbeispiele (Beispiel 25 ist ein Referenzbeispiel) zur Ausführungsform des Kombinationspräparats für Probanden mit Autoimmunthyreoiditis, wobei die Mengenangaben in mg/Kapsel erfolgen (ausgenommen Spalte 3).

| **Wirkstoff** | **24** | **24 (in Gew-%)** | **25** | **26** |
|---|---|---|---|---|
| Vitamin C | 100 | 32,99088792 | 100 | 120 |
| Vitamin E | 20 | 6,598177583 | 20 | 30 |
| Vitamin B1 | 3 | 0,989726638 | 3 | 4 |
| Vitamin B2 | 3 | 0,989726638 | 3 | 2 |
| Vitamin B5 | 12 | 3,95890655 | 12 | 15 |
| Vitamin B6 | 4 | 1,319635517 | 4 | 5 |
| Vitamin B12 | 0,009 | 0,00296918 | 0,009 | 0,01 |
| Vitamin B9 | 0,8 | 0,263927103 | 0,8 | 0,8 |
| Vitamin D3 | 0,005 | 0,001649544 | 0,005 | 0,01 |
| Niacin | 35 | 11,54681077 | - | 15 |
| Vitamin B7 | 0,2 | 0,065981776 | 0,2 | 0,3 |
| Zink | 5 | 1,649544396 | - | 6 |
| Selen | 0,1 | 0,032990888 | 0,2 | 0,05 |
| Magnesium | 100 | 32,99088792 | - | 200 |
| Coenzym Q10 | 20 | 6,598177583 | 0,1 | 0,05 |
| Eisen | 7,5 | 2,474316594 | - | 14 |
| Nicotinamid | - | - | - | 20 |
| Calcium | - | - | - | 125 |

Die oben genannten Ausführungsbeispiele zeigen besonders gute Wirkungen bei Frauen mit Autoimmunthyreoiditis und Kinderwunsch, sowohl bei Spontankonzeption als auch im Rahmen reproduktionsmedizinscher Behandlung. Die oben genannten Ausführungsvarianten 24 bis 26 enthalten eine Kombination von Mikronährstoffen, die die gesunde Entwicklung der Eizellen unterstützt und den Krankheitsverlauf bei einer Autoimmunthyreoiditis begünstigt. Mit dem Kombinationspräparat gemäß Ausführungsbeispiel 24 werden bessere Ergebnisse als mit einem Kombinationspräpart gemäß Beispiel 25 und 26 erzielt. Dieser Vitalstoffkomplex fördert die Fruchtbarkeit und trägt zur gesunden Entwicklung des Babys in den ersten Wochen bei.

Vorzugsweise sind die Ausführungsvarianten des Kombinationspräparats frei von Gelatine und Laktose.

Alle Ausführungsvarianten des Kombinationspräparats sollten einmal täglich in Form einer Kapsel oral eingenommen werden. Die empfohlene Mindesteinnahmedauer beträgt 1 bis 3 Monate. Das Präparat sollte dann bei Eintreten einer Schwangerschaft zumindest bis in die 12. Schwangerschaftswoche weiterhin eingenommen werden.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mitumfasst sind, d.h. sämtliche Teilbereich beginnen mit einer unteren Grenze von 1 oder größer und enden bei einer oberen Grenze von 10 oder weniger, z.B. 1 bis 1,7, oder 3,2 bis 8,1 oder 5,5 bis 10.

Die Ausführungsbeispiele zeigen mögliche Ausführungsvarianten des Kombinationspräparats, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der beschriebenen Ausführungsvariante möglich sind, vom Schutzumfang mit umfasst.

Die den eigenständigen erfinderischen Lösungen zugrunde liegende Aufgabe kann der Beschreibung entnommen werden.

## Patentansprüche

1. Zusammensetzung zur Verwendung in der Behandlung weiblicher Infertilität bei Patientinnen mit Autoimmunthyreoiditis umfassend zumindest die Wirkstoffe C-Vitamin, E-Vitamine, B1-Vitamin, B2-Vitamin, B3-Vitamin, B5-Vitamin, B6-Vitamine, B7-Vitamin, B9-Vitamin, B12-Vitamine, D-Vitamine, Zink, Magnesium, Eisen, Selen und/oder eine Selenverbindung, und zumindest eine Ubiquinon-10-Komponente, **dadurch gekennzeichnet, dass** sie jodfrei ist.

2. Zusammensetzung zur Verwendung nach Anspruch 1, **dadurch gekennzeichnet, dass** C-Vitamine in Mengen von 4 bis 60 Gew.-%, insbesondere 10 bis 45 Gew.-%, vorzugsweise 19 bis 33 Gew.-%, E-Vitamine in Mengen von 1 bis 15 Gew.-%, insbesondere 2 bis 10 Gew.-%, vorzugsweise 3 bis 6 Gew.-%, B1-Vitamine in Mengen von 0,1 bis 3 Gew.-%, insbesondere 0,2 bis 2 Gew.-%, vorzugsweise 0,3 bis 0,9 Gew.-%, B2-Vitamine in Mengen von 0,1 bis 3 Gew.-%, insbesondere 0,2 bis 2 Gew.-%, vorzugsweise 0,3 bis 0,9 Gew.-%, B5-Vitamine in Mengen von 0,5 bis 10 Gew.-%, insbesondere 1 bis 5 Gew.-%, vorzugsweise 1,2 bis 4 Gew.-%, B6-Vitamine in Mengen von 0,1 bis 5 Gew.-%, insbesondere 0,5 bis 3 Gew.-%, vorzugsweise 0,8 bis 1,3 Gew.-%, B9-Vitamine in Mengen von 0,01 bis 1 Gew.-%, insbesondere 0,05 bis 0,5 Gew.-%, vorzugsweise 0,1 bis 0,3 Gew.-%, B12-Vitamine in Mengen von 0,0003 bis 0,01 Gew.-%, insbesondere 0,0009 bis 0,008 Gew.-%, vorzugsweise 0,001 bis 0,01 Gew.-%, D-Vitamine in Mengen von 0,0002 bis 0,01 Gew.-%, insbesondere 0,0008 bis 0,008 Gew.-%, vorzugsweise 0,001 bis 0,01 Gew.-%, eine Ubiquinon-10-Komponente in Mengen von 2 bis 25 Gew.-%, insbesondere 5 bis 15 Gew.-%, vorzugsweise 6 bis 12 Gew.-%, B7-Vitamine in Mengen von 0,001 bis 0,5 Gew.-%, insbesondere 0,007 bis 0,1 Gew.-%, vorzugsweise 0,01 bis 0,08, Niacin in Mengen von 1 bis 20 Gew.-%, insbesondere 3 bis 15 Gew.-%, vorzugsweise 5 bis 11 Gew.-%, Zink und/oder einer Zinkverbindung in Mengen von 0,2 bis 5 Gew.-%, insbesondere 1 bis 4,5 Gew.-%, vorzugsweise 1,5 bis 3,5 Gew.-%, Magnesium und/oder einer Magnesiumverbindung in Mengen von 10 bis 50 Gew.-%, insbesondere 15 bis 40 Gew.-%, vorzugsweise 25 bis 35 Gew.-%, Eisen und/oder eine Eisenverbindung in Mengen von 0,5 bis 10 Gew.-%, insbesondere 1 bis 5 Gew.-%, vorzugsweise 1,5 bis 3 Gew.-%, und Selen und/oder eine Selenverbindung in Mengen von 0,001 bis 0,1 Gew.-%, insbesondere 0,007 bis 0,08 Gew.-%, vorzugsweise 0,01 bis 0,05 Gew.-%, bezogen auf das Gesamtgewicht der therapeutisch wirksamen Wirkstoffe des Kombinationspräparats enthalten sind.

3. Zusammensetzung zur Verwendung nach Anspruch 1 oder 2, **dadurch gekennzeichnet, dass** Calcium und/oder eine Calciumverbindung und/oder Nikotinsäureamid enthalten ist.

4. Zusammensetzung zur Verwendung nach Anspruch 3, **dadurch gekennzeichnet, dass** Calcium und/oder eine Calciumverbindung in Mengen von 5 bis 50 Gew.-%, insbesondere 15 bis 40 Gew.-%, vorzugsweise 20 bis 33 Gew.-%, und/oder Nikotinsäureamid in Mengen von 1 bis 15 Gew.-%, insbesondere 2 bis 10 Gew.-%, vorzugsweise 3 bis 6 Gew.-%, bezogen auf das Gesamtgewicht der therapeutisch wirksamen Wirkstoffe des Kombinationspräparats enthalten ist.

5. Zusammensetzung zur Verwendung nach einem der Ansprüche 1 bis 4, **dadurch gekennzeichnet, dass** weitere Spurenelemente ausgewählt aus einer Gruppe umfassend Kupfer und/oder eine Kupferverbindung, Molybdän und/oder eine Molybdänverbindung, Chrom und/oder eine Chromverbindung, Phosphor und/oder eine Phosphorverbindung, Mangan und/oder eine Manganverbindung oder einer Mischung aus zwei oder mehreren dieser Verbindungen enthalten ist.

6. Zusammensetzung zur Verwendung nach Anspruch 5, **dadurch gekennzeichnet, dass** Spurenelemente, ausgewählt aus einer Gruppe umfassend Kupfer und/oder eine Kupferverbindung in Mengen von 0,05 bis 3 Gew.-%, insbesondere 0,08 bis 1 Gew.-%, vorzugsweise 0,1 bis 0,5 Gew.-%, Molybdän und/oder eine Molybdänverbindung in Mengen von 0,0001 bis 0,5 Gew.-%, insbesondere 0,001 bis 0,1 Gew.-%, vorzugsweise 0,002 bis 0,05 Gew.-%, Chrom und/oder eine Chromverbindung in Mengen von 0,0001 bis 0,5 Gew.-%, insbesondere 0,001 bis 0,1 Gew.-%, vorzugsweise 0,002 bis 0,05 Gew.-%, Phosphor und/oder eine Phosphorverbindung in Mengen von 0,1 bis 30 Gew.-%, insbesondere 1 bis 20 Gew.-%, vorzugsweise 10 bis 15 Gew.-%, Mangan und/oder eine Manganverbindung in Mengen von 0,05 bis 3 Gew.-%, insbesondere 0,08 bis 1 Gew.-%, vorzugsweise 0,1 bis 0,5 Gew.-%, bezogen auf das Gesamtgewicht der therapeutisch wirksamen Wirkstoffe des Kombinationspräparats enthalten sind.

7. Zusammensetzung zur Verwendung nach einem der Ansprüche 1 bis 6, **dadurch gekennzeichnet, dass** Vorstufen von A-Vitaminen, insbesondere, α- und β-Carotin, α- und β-Cryptoxanthin und Lycopin, und/oder K-Vitamine, insbesondere K1, K2, K3 bezogen auf das Gesamtgewicht der therapeutisch wirksamen Wirkstoffe des Kombinationspräparats enthalten sind.

8. Zusammensetzung zur Verwendung nach Anspruch 7, **dadurch gekennzeichnet, dass** Vorstufen von A-Vitaminen, insbesondere, α- und β-Carotin, α- und β-Cryptoxanthin und Lycopin, in Mengen von 0,0001 bis 0,05 Gew.-%, insbesondere 0,0002 bis 0,001 Gew.-%, vorzugsweise 0,0003 bis 0,0006 Gew.-%, und/oder K-Vitamine, insbesondere K1, K2, K3 in Mengen von 0,05 bis 3 Gew.-%, insbesondere 0,08 bis 1 Gew.-%, vorzugsweise 0,1 bis 0,5 Gew.-%, bezogen auf das Gesamtgewicht der therapeutisch wirksamen Wirkstoffe des Kombinationspräparats enthalten sind.

9. Zusammensetzung zur Verwendung nach einem der Ansprüche 1 bis 8, **dadurch gekennzeichnet, dass** Omega-3-Fettsäuren, insbesondere DHA und/oder EPA, enthalten sind.

10. Zusammensetzung zur Verwendung nach Anspruch 9, **dadurch gekennzeichnet, dass** Omega-3-Fettsäuren, insbesondere DHA und/oder EPA, in Mengen von 2 bis 70 Gew.-%, insbesondere 10 bis 50 Gew.-%, vorzugsweise 20 bis 40 Gew.-%, bezogen auf das Gesamtgewicht der therapeutisch wirksamen Wirkstoffe des Kombinationspräparats enthalten sind.

11. Zusammensetzung zur Verwendung nach einem der Ansprüche 1 bis 10, **dadurch gekennzeichnet, dass** Probiotika in Mengen von 0,1 bis 10 Gew.-%, insbesondere 0,5 bis 5 Gew.-%, vorzugsweise 1 bis 3 Gew.-%, bezogen auf das Gesamtgewicht der therapeutisch wirksamen Wirkstoffe des Kombinationspräparats enthalten sind.

## Claims

1. A composition for use in the treatment of infertility in female patients with autoimmune thyroiditis, comprising at least the substances vitamin C, vitamin E, vitamin B1, vitamin B2, vitamin B3, vitamin B5, vitamin B6, vitamin B7, vitamin B9, B12 vitamins, D vitamins, zinc, magnesium, iron, selenium and/or a selenium compound, and at least one ubiquinon 10 component, **characterized in that** it is free from iodine.

2. The composition for use as claimed in claim 1, **characterized in that** it contains C vitamins in quantities of 4% to 60% by weight, in particular 10% to 45% by weight, preferably 19% to 33% by weight, E vitamins in quantities of 1% to 15% by weight, in particular 2% to 10% by weight, preferably 3% to 6% by weight, B1 vitamins in quantities of 0.1% to 3% by weight, in particular 0.2% to 2% by weight, preferably 0.3% to 0.9% by weight, B2 vitamins in quantities of 0.1% to 3% by weight, in particular 0.2% to 2% by weight, preferably 0.3% to 0.9% by weight, B5 vitamins in quantities of 0.5% to 10% by weight, in particular 1% to 5% by weight, preferably 1.2% to 4% by weight, B6 vitamins in quantities of 0.1% to 5% by weight, in particular 0.5% to 3% by weight, preferably 0.8% to 1.3% by weight, B9 vitamins in quantities of 0.01% to 1% by weight, in particular 0.05% to 0.5% by weight, preferably 0.1% to 0.3% by weight, B12 vitamins in quantities of 0.0003% to 0.01% by weight, in particular 0.0009% to 0.008% by weight, preferably 0.001% to 0.01% by weight, D vitamins in quantities of 0.0002% to 0.01% by weight, in particular 0.0008% to 0.008% by weight, preferably 0.001% to 0.01% by weight, a ubiquinon 10 component in quantities of 2% to 25% by weight, in particular 5% to 15% by weight, preferably 6% to 12% by weight, B7 vitamins in quantities of 0.001% to 0.5% by weight, in particular 0.007% to 0.1% by weight, preferably 0.01% to 0.08% by weight, niacin in quantities of 1% to 20% by weight, in particular 3% to 15% by weight, preferably 5% to 11% by weight, zinc and/or a zinc compound in quantities of 0.2% to 5% by weight, in particular 1% to 4.5% by weight, preferably 1.5% to 3.5% by weight, magnesium and/or a magnesium compound in quantities of 10% to 50% by weight, in particular 15% to 40% by weight, preferably 25% to 35% by weight, iron and/or an iron compound in quantities of 0.5% to 10% by weight, in particular 1% to 5% by weight, preferably 1.5% to 3% by weight, and selenium and/or a selenium compound in quantities of 0.001% to 0.1% by weight, in particular 0.007% to 0.08% by weight, preferably 0.01% to 0.05% by weight, with respect to the total weight of the therapeutically effective substances of the combination product.

3. The composition for use as claimed in claim 1 or claim 2, **characterized in that** it contains calcium and/or a calcium compound and/or nicotinamide.

4. The composition for use as claimed in claim 3, **characterized in that** it contains calcium and/or a calcium compound in quantities of 5% to 50% by weight, in particular 15% to 40% by weight, preferably 20% to 33% by weight, and/or nicotinamide in quantities of 1% to 15% by weight, in particular 2% to 10% by weight, preferably 3% to 6% by weight, with respect to the total weight of the therapeutically effective substances of the combination product.

5. The composition for use as claimed in one of claims 1 to 4, **characterized in that** it contains further trace elements selected from the group comprising copper and/or a copper compound, molybdenum and/or a molybdenum compound, chromium and/or a chromium compound, phosphorus and/or a phosphorus compound, manganese and/or a manganese compound or a mixture of two of more of these compounds.

6. The composition for use as claimed in claim 5, **characterized in that** it contains the trace elements selected from a group comprising copper and/or a copper compound in quantities of 0.05% to 3% by weight, in particular 0.08% to 1% by weight, preferably 0.1% to 0.5% by weight, molybdenum and/or a molybdenum compound in quantities of 0.0001% to 0.5% by weight, in particular 0.001% to 0.1% by weight, preferably 0.002% to 0.05% by weight, chromium and/or a chromium compound in quantities of 0.0001% to 0.5% by weight, in particular 0.001% to 0.1% by weight, preferably 0.002% to 0.05% by weight, phosphorus and/or a phosphorus compound in quantities of 0.1% to 30% by weight, in particular 1% to 20% by weight, preferably 10% to 15% by weight, manganese and/or a manganese compound in quantities of 0.05% to 3% by weight, in particular 0.08% to 1% by weight, preferably 0.1% to 0.5% by weight, with respect to the total weight of the therapeutically effective substances of the combination product.

7. The composition for use as claimed in one of claims 1 to 6, **characterized in that** it contains precursors of A vitamins, in particular α- and β- carotene, α- and β-cryptoxanthin and lycopene, and/or K vitamins, in particular K1, K2, K3, with respect to the total weight of the therapeutically effective substances of the combination product.

8. The composition for use as claimed in claim 7, **characterized in that** it contains precursors of A vitamins, in particular α- and β- carotene, α- and β-cryptoxanthin and lycopene, in quantities of 0.0001% to 0.05% by weight, in particular 0.0002% to 0.001% by weight, preferably 0.0003% to 0.0006% by weight, and/or K vitamins, in particular K1, K2, K3, in quantities of 0.05% to 3% by weight, in particular 0.08% to 1% by weight, preferably 0.1% to 0.5% by weight, with respect to the total weight of the therapeutically effective substances of the combination product.

9. The composition for use as claimed in one of claims 1 to 8, **characterized in that** it contains omega-3 fatty acids, in particular DHA and/or EPA.

10. The composition for use as claimed in claim 9, **characterized in that** it contains omega-3 fatty acids, in particular DHA and/or EPA, in quantities of 2% to 70% by weight, in particular 10% to 50% by weight, preferably 20% to 40% by weight, with respect to the total weight of the therapeutically effective substances of the combination product.

11. The composition for use as claimed in one of claims 1 to 10, **characterized in that** it contains probiotics in quantities of 0.1% to 10% by weight, in particular 0.5% to 5% by weight, preferably 1% to 3% by weight, with respect to the total weight of the therapeutically effective substances of the combination product.

## Revendications

1. Composition destinée à être utilisée pour le traitement de l'infertilité féminine chez les patientes présentant une thyroïdite auto-immune, comprenant au moins les principes actifs vitamine C, vitamines E, vitamine B1, vitamine B2, vitamine B3, vitamine B5, vitamines B6, vitamine B7, vitamine B9, vitamines B12, vitamines D, zinc, magnésium, fer, sélénium et/ou un composé du sélénium, et au moins un composant ubiquinone-10, **caractérisée en ce qu'**elle est exempte d'iode.

2. Composition pour l'utilisation selon la revendication 1, **caractérisée en ce qu'**elle contient des vitamines C en des quantités de 4 à 60 % en poids, en particulier de 10 à 45 % en poids, de préférence de 19 à 33 % en poids, des vitamines E en des quantités de 1 à 15 % en poids, en particulier de 2 à 10 % en poids, de préférence de 3 à 6 % en poids, des vitamines B1 en des quantités de 0,1 à 3 % en poids, en particulier de 0,2 à 2 % en poids, de préférence de 0,3 à 0,9 % en poids, des vitamines B2 en des quantités de 0,1 à 3 % en poids, en particulier de 0,2 à 2 % en poids, de préférence de 0,3 à 0,9 % en poids, des vitamines B5 en des quantités de 0,5 à 10 % en poids, en particulier de 1 à 5 % en poids, de préférence de 1,2 à 4 % en poids, des vitamines B6 en des quantités de 0,1 à 5 % en poids, en particulier de 0,5 à 3 % en poids, de préférence de 0,8 à 1,3 % en poids, des vitamines B9 en des quantités de 0,01 à 1 % en poids, en particulier de 0,05 à 0,5 % en poids, de préférence de 0,1 à 0,3 % en poids, des vitamines B12 en des quantités de 0,0003 à 0,01 % en poids, en particulier de 0,0009 à 0,008 % en poids, de préférence de 0,001 à 0,01 % en poids, des vitamines D en des quantités de 0,0002 à 0,01 % en poids, en particulier de 0,0008 à 0,008 % en poids, de préférence de 0,001 à 0,01 % en poids, un composant ubiquinone-10 en des quantités de 2 à 25 % en poids, en particulier de 5 à 15 % en poids, de préférence de 6 à 12 % en poids, des vitamines B7 en des quantités de 0,001 à 0,5 % en poids, en particulier de 0,007 à 0,1 % en poids, de préférence de 0,01 à 0,08 % en poids, de la niacine en des quantités de 1 à 20 % en poids, en particulier de 3 à 15 % en poids, de préférence de 5 à 11 % en poids, du zinc et/ou un composé du zinc en des quantités de 0,2 à 5 % en poids, en particulier de 1 à 4,5 % en poids, de préférence de 1,5 à 3,5 % en poids, du magnésium et/ou un composé du magnésium en des quantités de 10 à 50 % en poids, en particulier de 15 à 40 % en poids, de préférence de 25 à 35 % en poids, du fer et/ou un composé du fer en des quantités de 0,5 à 10 % en poids, en particulier de 1 à 5 % en poids, de préférence de 1,5 à 3 % en poids, et du sélénium et/ou un composé du sélénium en des quantités de 0,001 à 0,1 % en poids, en particulier de 0,007 à 0,08 % en poids, de préférence de 0,01 à 0,05 % en poids, par rapport au poids total des principes actifs à activité thérapeutique de la préparation en association.

3. Composition pour l'utilisation selon la revendication 1 ou 2, **caractérisée en ce qu'**elle contient du calcium et/ou un composé du calcium et/ou du nicotinamide.

4. Composition pour l'utilisation selon la revendication 3, **caractérisée en ce qu'**elle contient du calcium et/ou un composé du calcium en des quantités de 5 à 50 % en poids, en particulier de 15 à 40 % en poids, de préférence de 20 à 33 % en poids, et/ou du nicotinamide en des quantités de 1 à 15 % en poids, en particulier de 2 à 10 % en poids, de préférence de 3 à 6 % en poids, par rapport au poids total des principes actifs à activité thérapeutique de la préparation en association.

5. Composition pour l'utilisation selon l'une des revendications 1 à 4, **caractérisée en ce qu'**elle contient des oligoéléments supplémentaires choisis dans un groupe comprenant le cuivre et/ou un composé du cuivre, le molybdène et/ou un composé du molybdène, le chrome et/ou un composé du chrome, le phosphore et/ou un composé du phosphore, le manganèse et/ou un composé du manganèse, ou un mélange d'au moins deux de ces composés.

6. Composition pour l'utilisation selon la revendication 5, **caractérisée en ce qu'**elle contient des oligoéléments choisis dans un groupe comprenant le cuivre et/ou un composé du cuivre en des quantités de 0,05 à 3 % en poids, en particulier de 0,08 à 1 % en poids, de préférence de 0,1 à 0,5 % en poids, le molybdène et/ou un composé du molybdène en des quantités de 0,0001 à 0,5 % en poids, en particulier de 0,001 à 0,1 % en poids, de préférence de 0,002 à 0,05 % en poids, le chrome et/ou un composé du chrome en des quantités de 0,0001 à 0,5 % en poids, en particulier de 0,001 à 0,1 % en poids, de préférence de 0,002 à 0,05 % en poids, le phosphore et/ou un composé du phosphore en des quantités de 0,1 à 30 % en poids, en particulier de 1 à 20 % en poids, de préférence de 10 à 15 % en poids, le manganèse et/ou un composé du manganèse en des quantités de 0,05 à 3 % en poids, en particulier de 0,08 à 1 % en poids, de préférence de 0,1 à 0,5 % en poids, par rapport au poids total des principes actifs à activité thérapeutique de la préparation en association.

7. Composition pour l'utilisation selon l'une des revendications 1 à 6, **caractérisée en ce qu'**elle contient des précurseurs de vitamines A, en particulier les carotènes α et β, les cryptoxanthines α et β et la lycopine, et/ou des vitamines K, en particulier K1, K2 et K3, par rapport au poids total des principes actifs à activité thérapeutique de la préparation en association.

8. Composition pour l'utilisation selon la revendication 7, **caractérisée en ce qu'**elle contient des précurseurs de vitamines A, en particulier les carotènes α et β, les cryptoxanthines α et β et la lycopine, en des quantités de 0,0001 à 0,05 % en poids, en particulier de 0,0002 à 0,001 % en poids, de préférence de 0,0003 à 0,0006 % en poids, et/ou des vitamines K, en particulier K1, K2 et K3, en des quantités de 0,05 à 3 % en poids, en particulier de 0,08 à 1 % en poids, de préférence de 0,1 à 0,5 % en poids, par rapport au poids total des principes actifs à activité thérapeutique de la préparation en association.

9. Composition pour l'utilisation selon l'une des revendications 1 à 8, **caractérisée en ce qu'**elle contient des acides gras oméga-3, en particulier le DHA et/ou l'EPA.

10. Composition pour l'utilisation selon la revendication 9, **caractérisée en ce qu'**elle contient des acides gras oméga-3, en particulier le DHA et/ou l'EPA, en des quantités de 2 à 70 % en poids, en particulier de 10 à 50 % en poids, de préférence de 20 à 40 % en poids, par rapport au poids total des principes actifs à activité thérapeutique de la préparation en association.

11. Composition pour l'utilisation selon l'une des revendications 1 à 10, **caractérisée en ce qu'**elle contient des probiotiques en des quantités de 0,1 à 10 % en poids, en particulier de 0,5 à 5 % en poids, de préférence de 1 à 3 % en poids, par rapport au poids total des principes actifs à activité thérapeutique de la préparation en association.
